# EUROPEAN PATENT APPLICATION

(11) **EP 4 361 254 A1**
(43) Date of publication of application: **01.05.2024**
(21) Application number: 22828514.4
(22) Date of filing: 23.06.2022
(51) Int. Cl.: C12N 5/07, C12N 1/12

(54) **METHOD FOR PRODUCING COMPOSITION FOR CULTURING ANIMAL CELLS, COMPOSITION FOR CULTURING ANIMAL CELLS OBTAINED BY SAID METHOD, AND METHOD FOR CULTURING ANIMAL CELLS USING SAID COMPOSITION FOR CULTURING ANIMAL CELLS**

(30) Priority: 23.06.2021 JP 2021104448
(71) Applicant: Tokyo Women's Medical University, Tokyo 162-8666 (JP)
(72) Inventor: SHIMIZU, Tatsuya, Tokyo 162-8666 (JP); HARAGUCHI, Yuji, Tokyo 162-8666 (JP); YAMANAKA, Kumiko, Tokyo 162-8666 (JP); HARA, Michikazu, Tokyo 152-8550 (JP); KITA, Yusuke, Tokyo 152-8550 (JP)
(74) Representative: Kransell & Wennborg KB
(86) International application number: PCT/JP2022/025179
(87) International publication number: WO 2022/270598

(57) **Abstract**

The present invention provides a method for producing a composition for culturing animal cells, wherein the method includes: (1) a step in which an algae is mixed with a solid acid catalyst and an algae extract is obtained by heating; and (2) a step in which the algae extract is added to a medium for culturing animal cells and the concentration of the algae extract is adjusted. The present invention also provides a recycling/culturing method for algae and animal cells including: (i) a step in which a waste liquid (a first waste liquid) previously used to culture animal cells is used to culture algae; (ii) a step in which the algae is collected, mixed with a solid catalyst, and heated, and an algae extract is thereby obtained; (iii) a step in which the algae extract is added to the waste liquid (a second waste liquid) previously used to culture algae in (i), the concentration of the algae extract is adjusted, and a composition for culturing animal cells is produced; and (iv) a step in which animal cells are cultured using the composition for culturing animal cells.

## Description

### FIELD

The present invention relates to a production method for a composition for culturing animal cells, to a composition for culturing animal cells obtained by it and to a method for culturing animal cells using it.

### BACKGROUND

Mammalian cells are used in a diverse range of research and industrial fields including medicine, pharmacy, science and engineering, agriculture and veterinary medicine. Research using mammalian cell culture technologies has led to a large number of disease causes being elucidated, and to the development of treatment methods for them. Biomedicines, antibody drugs and vaccines produced from mammalian cells are used for treatment and prevention of a large host of diseases. Cultured cells have also contributed in a major way to development in basic academic fields such as cell biology, biochemistry, genetics, physiology and molecular biology. More recently, cultured muscle cells have been used as cell sources for "cultured meat". The protein production efficiency for "cultured meat" is much higher than by production via conventional livestock breeding, and according to published reports, since meat production has traditionally depended on livestock breeding, switching to cultured meat production systems can result in reduction of up to 99% for utilization of land, 96% for water consumption, 96% for greenhouse gas emissions and 45% for energy consumption. It is believed, therefore, that food production systems allowing continuous meat production using cultured cells will become more widely popular in the future. Future use of cultured mammalian cells as a food source is expected to require very large amounts of cells.

Since culture media are essential for culturing of mammalian cells, mass quantities of culture media are even now being used for cell research. Such culture media contain various types of nutrients which consist mainly of glucose and amino acids. The nutrients are derived from cereals or heterotrophic microorganisms, the latter also requiring nutrients derived from cereals. Chemical fertilizers and agricultural chemicals are necessary in large amounts for cereal cultivation, which in turn requires massive amounts of energy, results in generation of more greenhouse gases (GHG) and causes environmental pollution. Cereal production is also significantly influenced by environmental effects such as global warming and environmental pollution.

At the current time, over 150,000 species of algae have been registered in AlgaeBase (http://www.algaebase.org/), with the number continuing to be updated on a daily basis. Numerous different algae properties exist because of the huge variety of their species, ranging from species that are able to grow in environments of either purified water or seawater, or both, to basophilic and alkaliphilic species. A wide variety of uses are currently under development to take advantage of those properties. Such development includes drainage treatment utilizing the nitrogen assimilation properties of microalgae, and fixation of carbon dioxide gas generated by combustion of fossil fuels (NPLs 1 to 3).

Microalgae synthesize nutrients such as carbohydrates (saccharides) and lipids from carbon dioxide by photosynthesis, and produce amino acids using nitrogen gas/ammonia/nitrates. Microalgae also efficiently produce various nutrients by utilizing solar energy and inorganic materials. The nutrients produced by microalgae are currently being actively utilized in a variety of fields including the energy and food industries. For example, lipids extracted from microalgae are considered promising as an alternative energy source to petroleum (NPL 4). Glucose extracted from microalgae is also utilized as a nutrient for bioethanol-producing yeast (NPLs 5 to 6). Nutrients containing amino acids produced by microalgae are also used in nutritional supplements, pet foods and soil fertilizers (NPLs 7 to 10). It is generally thought that microalgae are the photosynthetic organisms that synthesize nutrients most efficiently without residue (NPL 11).

Culturing of cells *in vitro* often requires human, fetal calf, cow neonatal, horse, chicken or rabbit serum in addition to synthetic medium containing amino acids, vitamins and minerals. Serum contains factors necessary for cell growth, such as growth factors, which are often essential for growth of the cells. However, because serum is unstable and may differ in quality depending on the lot, while also being problematically expensive, there is a need to find alternative substances. In light of these problems, PTLs 1 to 3 describe microalgal extract that can partially substitute for the function of serum in cell culturing. However, these publications do not mention that cells can be cultured by mixing of a microalgal extract with synthetic media (such as inorganic salt medium) that lack components such as amino acids (especially glutamine) and vitamins that are essential for cell culturing, nor do they teach that these can completely substitute for serum or added factors for serum-free culture.

It has been reported that animal cells can be cultured in media (for example, media lacking amino acids and/or vitamins) with addition of algae extract obtained by hydrolysis of microalgae using liquid acid (PTL 4). However, the potential for such extract to completely substitute for serum or added factors for serum-free culture it is not mentioned.

Moreover, while it is mentioned that algae-derived amino acids (such as mycosporin-like amino acids (PTL 5)), or sulfated polysaccharides (especially fucodyne (PTL 6)) exhibit cell proliferation effects, it nowhere states that they can completely substitute for serum or added factors for serum-free culture.

Techniques have been reported which use solid acids as hydrolysis catalysts for obtaining monosaccharide-containing saccharified solutions from marine algae biomass or plant-based materials, but cell culturing with the saccharified solutions obtained by such techniques has not been disclosed (PTLs 7 and 8).

### [CITATION LIST]

### [PATENT LITERATURE]

[PTL 1] Japanese Unexamined Patent Publication SHO No. 57-65179
[PTL 2] Japanese Unexamined Patent Publication SHO No. 57-206384
[PTL 3] Japanese Unexamined Patent Publication SHO No. 60-186280
[PTL 4] International Patent Publication No. WO2021/066113
[PTL 5] Japanese Unexamined Patent Publication No. 2016-216750
[PTL 6] International Patent Publication No. WO00/62785
[PTL 7] Japanese Unexamined Patent Publication No. 2014-36589
[PTL 8] Japanese Unexamined Patent Publication No. 2019-199462

### [NON PATENT LITERATURE]

[NPL 1] L. Wang, M. Min, Y. Li, P. Chen, Y. Chen, et al., Cultivation of green algae Chlorella sp. in different wastewaters from municipal wastewater treatment plant. Appl. Biochem. Biotechnol. 162 (2010) 1174-1186.
[NPL 2] F. Rossi, E.J. Olguin, L. Diels, R. De Philippis, Microbial fixation of CO2 in water bodies and in drylands to combat climate change, soil loss and desertification. N. Biotechnol. 32 (2015) 109-120.
[NPL 3] J. Cheng, Y Zhu, Z. Zhang, W. Yang, Modification and improvement of microalgae strains for strengthening CO2 fixation from coal-fired flue gas in power plants. Bioresour. Technol. (2019) doi: 10.1016/j.biortech.2019.121850.
[NPL 4] I. Ajjawi, J. Verruto, M. Aqui, L.B. Soriaga, J. Coppersmith, et al., Lipid production in Nannochloropsis gaditana is doubled by decreasing expression of a single transcriptional regulator. Nat. Biotechnol. 35 (2017) 647-652.
[NPL 5] S.H. Ho, S.W. Huang, C.Y Chen, T. Hasunuma, A. Kondo, et al., Bioethanol production using carbohydrate-rich microalgae biomass as feedstock. Bioresour. Technol. 135 (2013) 191-198.
[NPL 6] G.E. Lakatos, K. Ranglova, J. C. Manoel, T. Grivalsky, J. Kopecky, et al., Bioethanol production from microalgae polysaccharides. Folia Microbiol. (Praha). (2019) doi: 10.1007/s 12223-019-00732-0.
[NPL 7] S. McCusker, P.R. Buff, Z. Yu, A.J Fascetti, Amino acid content of selected plant, algae and insect species: a search for alternative protein sources for use in pet foods. J. Nutr. Sci. 3 (2014)e39.
[NPL 8] G. Gutierrez-Salmean, L. Fabila-Castillo, G. Chamorro-Cevallos, Nutritional and toxicological aspects of Spirulina (Arthrospira). Nutr. Hosp. 32 (2015) 34-40.
[NPL 9] E.T. Alori, O.O. Babalola, Microbial inoculants for improving crop quality and human health in Africa. Front. Microbiol. 9 (2018) 2213.
[NPL 10] M.I. Khan, J. H. Shin, J. D. Kim, The promising future of microalgae: current status, challenges and optimization of a sustainable and renewable industry for biofuels, feed and other products. Microb. Cell Fact. 17 (2018) 36.
[NPL 11] A.K. Singh, M.P. Singh, Importance of algae as a potential source of biofuel. Cell Mol. Biol. (Noisy-le-grand). 60 (2014) 106-109.

### SUMMARY

### [TECHNICAL PROBLEM]

It is an object of the present invention to establish a novel cell culturing system that lowers the risk of environmental impact or environmental change.

### [SOLUTION TO PROBLEM]

The present inventors have conducted research and development based on examination of the aforementioned problem from many angles, in order to solve the problems described above. As a result it was found, surprisingly, that animal cells can be cultured in a medium (especially a medium substantially lacking serum and/or added factors for serum-free culture) containing added nutrients extracted from microalgae using a solid acid catalyst. Specifically, the invention encompasses the following.
[1] A method for producing a composition for culturing animal cells, comprising:
   (1) mixing algae with a solid acid catalyst and heating the mixture to obtain an algae extract; and
   (2) adding the algae extract to animal cell culturing medium and adjusting the concentration of the algae extract.
[2] The method according to [1] above, wherein the animal cell culturing medium is substantially free of serum and/or added factors for serum-free culture.
[3] The method according to [1] or [2] above, wherein the animal cell culturing medium is substantially free of glucose.
[4] The method according to any one of [1] to [3] above, wherein the animal cell culturing medium is substantially free of one or more amino acids selected from the group consisting of L-glutamine, L-arginine, L-cystine, glycine, L-histidine, L-isoleucine, L-leucine, L-lysine, L-methionine, L-phenylalanine, L-serine, L-threonine, L-tryptophan, L-tyrosine and L-valine.
[5] The method according to any one of [1] to [4] above wherein the animal cell culturing medium is substantially free of vitamins.
[6] The method according to any one of [1] to [5] above, wherein the animal cell culturing medium is an inorganic salt medium.
[7] The method according to any one of [1] to [6] above, wherein the algae are microalgae.
[8] The method according to any one of [1] to [7] above, wherein the algae are microalgae belonging to *Chlorella* sp.
[9] The method according to any one of [1] to [8] above, wherein the solid acid catalyst is a woody solid acid catalyst obtained by introducing a sulfo group into a carbide derived from a wood-based raw material for sulfonation, or a resin solid acid catalyst obtained by introducing a sulfo group into a phenol resin for sulfonation.
[10] The method according to any one of [1] to [9] above, wherein step (1) is carried out at 70°C to 150°C.
[11] The method according to any one of [1] to [10] above, wherein step (1) is carried out for 2 hours to 24 hours.
[12] A composition for culturing animal cells which is obtained by the method according to any one of [1] to [11] above.
[13] A method for culturing animal cells using a composition for culturing animal cells which is obtained by the method according to any one of [1] to [11] above.
[14] A recycling culture method for algae and animal cells, comprising:
   (i) culturing algae using a waste medium obtained from culturing animal cells (first waste medium);
   (ii) collecting the algae, mixing it with a solid acid catalyst and heating the mixture, to obtain an algae extract;
   (iii) adding the algae extract to the waste medium obtained from culturing the algae of step (i) (second waste medium) and adjusting the concentration of the algae extract to produce a composition for culturing animal cells; and
   (iv) using the composition for culturing animal cells for culturing of animal cells.
[15] A method for producing an algae extract for use in preparation of a composition for culturing animal cells, comprising:
   (1) mixing algae with a solid acid catalyst and heating the mixture to obtain an algae extract.
[16] The method according to [15] above, wherein the animal cell culturing medium is substantially free of serum and/or added factors for serum-free culture.
[17] The method according to [15] or [16] above, wherein the composition for culturing animal cells is substantially free of glucose.
[18] The method according to any one of [15] to [17] above, wherein the composition for culturing animal cells is substantially free of one or more amino acids selected from the group consisting of L-glutamine, L-arginine, L-cystine, glycine, L-histidine, L-isoleucine, L-leucine, L-lysine, L-methionine, L-phenylalanine, L-serine, L-threonine, L-tryptophan, L-tyrosine and L-valine.
[19] The method according to any one of [15] to [18] above wherein the composition for culturing animal cells is substantially free of vitamins.
[20] The method according to any one of [15] to [19] above, wherein the composition for culturing animal cells is an inorganic salt medium.
[21] The method according to any one of [15] to [20] above, wherein the algae are microalgae.
[22] The method according to any one of [15] to [21] above, wherein the algae are microalgae belonging to *Chlorella* sp..
[23] The method according to any one of [15] to [22] above, wherein the solid acid catalyst is a woody solid acid catalyst obtained by introducing a sulfo group into a carbide derived from a wood-based raw material for sulfonation or a resin solid acid catalyst obtained by introducing a sulfo group into a phenol resin for sulfonation.
[24] The method according to any one of [15] to [23] above, wherein step (1) is carried out at 70°C to 150°C.
[25] The method according to any one of [15] to [24] above, wherein step (1) is carried out for 2 hours to 24 hours.
[26] The method according to any one of [15] to [25] above, wherein step (1) is carried out under pressure.

### [ADVANTAGEOUS EFFECTS OF INVENTION]

According to the method of the invention, nutrients necessary for cell culturing can be easily and economically extracted from microalgae, and it is thereby possible to provide a novel cell culturing system that reduces environmental load.

### BRIEF DESCRIPTION OF DRAWINGS

Fig. 1 shows relative viable cell counts of primary bovine myoblasts cultured using glucose/serum-containing and -free DMEM.
Fig. 2 shows relative viable cell counts of primary bovine myoblasts cultured using glucose/serum-free DMEM with addition of algae extract obtained under different extraction conditions.
Fig. 3 shows relative viable cell counts of primary bovine myoblasts cultured using glucose/serum-free DMEM with addition of algae extract obtained under different extraction conditions.
Fig. 4 shows microscope images of primary bovine myoblasts cultured in different media.
Fig. 5 shows microscope images of primary bovine myoblasts cultured in different media.
Fig. 6 shows relative viable cell counts of primary bovine myoblasts cultured using glucose/serum-free DMEM with addition of algae extract obtained under different extraction conditions.
Fig. 7A shows animal muscle cell cultures obtained using solid acid-treated algae (*Chlorococcum*) extract (photomicrographs).
Fig. 7B shows animal muscle cell cultures obtained using solid acid-treated algae (*Chlorococcum*) extract (quantitative evaluation).

### DESCRIPTION OF EMBODIMENTS

Embodiments of the invention will now be explained with reference to the accompanying drawings as necessary. The embodiments serve merely for example, and the construction of the invention is not limited by the concrete constructions of the embodiments. All of the publications mentioned throughout the present specification are incorporated herein in their entirety by reference.

According to one embodiment, the invention provides a method for producing a composition for culturing animal cells, comprising:
(1) mixing algae with a solid acid catalyst and heating the mixture to obtain an algae extract; and
(2) mixing the algae extract with animal cell culturing medium that is substantially free of serum and/or added factors for serum-free culture.

According to another embodiment, the present invention provides a method for producing a composition for culturing animal cells, comprising:
(1) mixing algae with a solid acid catalyst and heating the mixture to obtain an algae extract; and
(2) adding the algae extract to animal cell culturing medium and adjusting the concentration of the algae extract.

The invention has been completed upon finding, surprisingly, that a composition for culturing animal cells that includes algae-derived components (algae extract) obtained by the method of the invention, even when being substantially free of serum and/or added factors for serum-free culture, exhibits an effect equivalent to or greater than medium for animal cells that contains serum or added factors for serum-free culture considered necessary for maintenance or proliferation of animal cells. The composition for culturing animal cells provided by the invention therefore does not require addition of serum or added factors for serum-free medium used in conventional culturing of animal cells. The present invention contributes to solve the problems with serum, in particular, which is expensive and differs in quality between lots. The concentration of the algae extract in the composition for culturing animal cells will differ depending on the extraction conditions for the algae extract, the type and amount of algae used as starting material, and the type of animal cells being cultured, but the algae extract may be used at 0.1 to 99% (v/v), 1 to 80% (v/v), 2 to 50% (v/v), 3 to 30% (v/v) or 5 to 25% (v/v), for example, with respect to the total volume of the composition for culturing animal cells, though this is not limitative.

As used herein, "serum" refers to the pale yellow liquid component appearing in supernatant when blood coagulates, and it includes factors necessary for cell proliferation, such as growth factors. The source is not particularly restricted and may be a human, cow fetus, cow neonate, horse, chicken or rabbit. The composition for culturing animal cells provided by the invention, even when including no serum, can be used to cause proliferation of animal cells at a level equivalent to or above that obtained when including serum.

As used herein, the phrase "added factors for serum-free culture" refers to factors that are commonly added instead of serum in serum-free medium and that cause proliferation (growth) of cells, such as insulin, transferrin, ethanolamine, sodium selenite, albumin, mercaptoethanol and prostaglandin, which are added to serum-free medium according to the cells of interest. The composition for culturing animal cells provided by the invention, even without including such added factors for serum-free culturing, can be used to cause proliferation of animal cells at a level equivalent to or above that obtained when including added factors for serum-free culturing.

As used herein, the term "solid acid catalyst" (also "solid acid") refers to a catalyst comprising an acid that is dispersed as a solid in a reaction mixture, examples of which include inorganic solid acids such as zeolite, alumina, silica, H-mordenite and niobic acid, and catalysts having acidic groups such as sulfone, carboxyl or phenolic hydroxyl groups introduced into an organic material such as a resin, (for example, strongly acidic ion exchangers such as AMBERLYST^{™}-15 and NATION^{™} NR-50), and solid acid catalysts prepared in powdered or granular form to increase the contact surface area may also be used. Using a solid acid for hydrolysis of algae eliminates the need for neutralization treatment which has been required when using liquid acids. This can suppress the increase in salt concentration that is caused by salts generated by neutralization treatment. A solid acid catalyst can also be recovered and reused, helping to reduce the environmental impact.

According to another embodiment, the solid acid catalyst to be used for the invention may be a woody solid acid catalyst obtained by introducing a sulfo group into a carbide derived from a wood-based raw material for sulfonation or a resin solid acid catalyst obtained by introducing a sulfo group into a phenol resin for sulfonation.

A woody solid acid catalyst to be used for this embodiment of the invention may be obtained by sulfonation, in which a wood-based raw material is carbonized under temperature conditions in which it does not burn up to produce a carbide, and sulfo groups (or sulfonate groups) are introduced. Examples of woody solid acid catalysts to be used include the solid acids disclosed in Japanese Patent No. 5528036 and J. Am. Chem. Soc 2008, vol. 130, No.38, 12787-12793.

A resin solid acid catalyst to be used for this embodiment of the invention may also be obtained by introduction of sulfo groups into a phenol resin raw material for sulfonation.

The animal cell culturing medium to be used for the invention may be an animal cell culturing medium that is also substantially free of one or more amino acids selected from the group consisting of L-glutamine, L-arginine, L-cystine, glycine, L-histidine, L-isoleucine, L-leucine, L-lysine, L-methionine, L-phenylalanine, L-serine, L-threonine, L-tryptophan, L-tyrosine and L-valine. Even though lacking amino acids that are included in conventional animal cell culturing media, the algae extract obtained according to the invention is able to substitute for the amino acids and to cause proliferation of animal cells.

According to another embodiment, the animal cell culturing medium to be used for the invention may be animal cell culturing medium that is substantially free of vitamins. Animal cell culturing medium that is substantially free of vitamins to be used for the invention may be medium that is substantially free of one or more vitamins selected from the group consisting of pantothenic acid, choline chloride, folic acid, *i*-inositol, niacinamide, pyridoxine, riboflavin, thiamine, and pharmaceutically acceptable salts of the same. Even though lacking vitamins that are included in conventional animal cell culturing media, the algae extract obtained according to the invention is able to substitute for the vitamins and to cause proliferation of animal cells.

According to yet another embodiment, the animal cell culturing medium to be used for the invention may be animal cell culturing medium that is substantially free of glucose, or it may be animal cell culturing medium that is also substantially free of pyruvic acid. Even though lacking glucose that is included in conventional animal cell culturing media, the algae extract obtained according to the invention is able to substitute for the glucose and to cause proliferation of animal cells.

As used herein, "substantially free of" means containing substantially none of the substance in question, but it does not mean containing absolutely none of the substance, and for example, glucose may be present at less than 10 mg/L (such as <10 mg/L, <5 mg/L or <1 mg/L) as the lower quantifiable limit, amino acids (such as L-glutamine) may be present at less than 0.1 mg/L as the lower quantifiable limit, or vitamins may be present at less than 1 µg/L as the lower quantifiable limit. For serum, it may be present at less than 1% (v/v) (such as less than 1% (v/v), less than 0.5% (v/v) or less than 0.1% (v/v)), and for added factors in serum-free culturing, they may be present at less than 1 µg/L (such as less than 1 µg/L, less than 0.5 µg/L or less than 0.1 µg/L).

Also as used herein, "algae" is a general term referring to organisms that produce oxygen by photosynthesis, with the exclusion of primarily land-inhabiting bryophytes, pteridophytes and spermatophytes. If given an environment necessary for photosynthesis, algae can produce oxygen and nutrients (such as glucose and amino acids) by themselves and proliferate. The present invention has been completed based on the finding that cells can be cultured in medium containing an added composition that comprises components extracted from algae.

The algae to be used for the invention, according to one embodiment, may be "microalgae" (also known as "unicellular algae"). As used herein, the term "microalgae" refers to algae where each consists of a single cell, also including forms with multiple microalgae individuals gathered together to form microalgae colonies, and which may be freshwater or marine, or stenohaline or euryhaline microalgae. Examples of microalgae include green algae in which chlorophyll a and b are the major pigments of the chloroplasts, unicellular blue-green algae (cyanobacteria) in which chlorophyll d is the major pigment, and unicellular red algae in which chlorophyll a and phycobilin proteins are the major pigments. More specific examples include the green algae *Chlamydomonas reinhardtii* of the class Chlorophyceae, order Chlamydomonadales (Japanese name: Chlamydomonas) (freshwater), *Dunaliella salina* of the order Dunalielles (Japanese name: Dunaliella) (marine), *Volvox carteri* of the order Volvocales (Japanese name Volvox) (freshwater), *Chlorococcum littorals* of the order Chlorococcales (marine), *Hydrodictyon reticulatum* (Japanese name: Amimidoro) (freshwater), *Pediastrum duplex* (Japanese name: Kunshoumo) (freshwater) and *Scenedesmus dimorphus* (Japanese name: Ikadamo) (freshwater) of the order Sphaeropleales, *Chlorella* sp. of the class Trebouxiophyceae, order Chlorellales (Japanese name: Chlorella) (for example, *Chlorella vulgaris* (freshwater), *Chlorella pyrenoidosa* (freshwater), *Chlorella ellipsoidea* (freshwater) and *Chlorella regularis* (freshwater)), and *Euglena gracilis* and *Euglena proxima* (Japanese name: Midorimushi) (freshwater) of the phylum Euglenozoa, class Euglenophyceae, order Euglena. Unicellular blue-green algae include *Acaryochloris marina* (marine), *Spirulina subsalsa* (freshwater) and *Arthrospira platensis* (freshwater) and *Synechococcus* sp. (marine or freshwater), of the phylum Cyanobacteria. Unicellular red algae include *Cyanidium caldarium* of the phylum Rhodophyta, class Cyanidiophyceae, order Cyanidiales (Japanese name Ideyukogome) (freshwater), and *Galdieria partita* of the phylum Rhodophyta, class Cyanidiophyceae, order Cyanidiales (freshwater). Unicellular axle algae include *Stichococcus* sp. of the phylum Chlorophyta, class Klebsormidiophyceae, order Klebsormidiales (marine). Unicellular ulvophyte algae include filamentous ulvophytes. The algae to be used for the invention are not limited to the algae mentioned above and may also be gene recombinant forms produced by genetic engineering of the aforementioned algae. For example, the algae to be used for the invention may be freshwater or marine and/or stenohaline or euryhaline microalgae, but are preferably marine and/or euryhaline microalgae, such as *Chlorococcum littorals,* or *Synechococcus* algae, although this is not limitative.

The algae used for the invention may be natural algae or algae grown by publicly known culturing methods.

Biopolymers such as proteins and polysaccharides generally undergo hydrolysis under heating (such as about 60°C to about 200°C, preferably about 80°C to about 180°C, more preferably about 70°C to about 150°C and even more preferably about 95°C to about 135°C), producing oligomers or monomers (peptides or amino acids, for proteins, and oligosaccharides or monosaccharides, for polysaccharides). According to the invention, therefore, the heating step may be carried out at about 60°C to about 200°C, preferably about 80°C to about 180°C, more preferably about 70°C to about 150°C and even more preferably about 95°C to about 135°C. The time for the heating step of the invention may be adjusted according to the conditions such as the type and amount of algae used, or the concentration, or the reaction mixture pH and temperature, and for example, it may be a time of about 30 minutes to about 48 hours, about 1 hour to about 36 hours, about 2 hours to about 24 hours, about 2.5 hours to about 12 hours or about 2.5 hours to about 7 hours. As used herein, the term "about" means a value in the range of ±10%, preferably ±5%, more preferably ±3% and even more preferably ±1%, of the value to which it pertains.

According to one embodiment, the algae used in step (1) may be supplied for drying treatment, such as freeze-drying, before heat treatment.

According to another embodiment, step (1) may be carried out under pressure. The term "under pressure" as used herein may be atmospheric pressure, including air pressure conditions higher than 1 atmosphere, such as 1.1 atmospheres or higher, 1.5 atmospheres or higher, 1.8 atmospheres or higher or 2 atmospheres or higher. For example, it may be 1.1 to 300 atmospheres, 1.5 to 200 atmospheres, 1.8 to 100 atmospheres, 2 to 50 atmospheres or 2 to 20 atmospheres. The pressure conditions may be conditions created using any apparatus or method, such as pressurized conditions produced with an autoclave.

According to yet another embodiment, the invention provides a composition for culturing animal cells obtained by the method described above.

According to yet another embodiment, the invention provides a method for culturing animal cells using a composition for culturing animal cells obtained by the method described above.

As used herein, "animal cells" includes cells from a vertebrate (mammal (or "mammalian animal"), bird, amphibian, reptile or fish), or cells from an invertebrate (such as a sea squirt, arthropod (crustacean (shrimp or crab, for example) or insect), an echinoderm (such as a sea urchin, sea cucumber or starfish), or a mollusk (such as shellfish, squid or octopus)). According to one embodiment, the animal cells to which the invention is to be applied may be cells of a vertebrate (for example, a mammal such as a human, monkey, cow, whale, bear, deer horse, pig, boar, sheep, rabbit, rat, mouse, hamster, goat, dog or cat, a bird such as a chicken, domesticated duck, goose, quail, wild duck or pheasant, an amphibian such as a frog, salamander or newt, a reptile such as a crocodile, lizard, snake, tortoise or terrapin, or a fish such as a tuna, salmon, trout, koi fish, shark, eel or puffer fish), an invertebrate (for example, a sea squirt, an arthropod (such as a crustacean (such as shrimp or crab) or an insect), an echinoderm (such as a sea urchin, sea cucumber or starfish), a mollusk (such as a shellfish, squid or octopus)), or primary cells, an established cell line or pluripotent stem cells (such as ES cells, ntES cells, Muse cells or iPS cells) or tissue stem cells (such as mesenchymal stem cells) derived from such animals, or cells obtained by inducing differentiation of such cells. The source of the biological tissue for the animal cells may be muscle (such as myoblasts), skin (such as fibroblasts or keratinocytes), liver (such as liver parenchymal cells), kidney (such as renal cells (for example, HEK293)), heart (such as myocardial cells), digestive system (such as oral mucosa cells, intestinal tract epithelial cells or parietal cells), the hematopoietic system (such as hematopoietic stem cells), reproductive tissue (such as ovary cells (for example, CHO cells), sperm cells or uterine epithelial cells), mucosa (such as epithelial cells), or bone tissue (such as osteoblasts or chondrocytes), as well as from any tissues other than these.

The composition for culturing animal cells to be provided by the invention may comprise one or more algae-derived components, such as two or more algae-derived components, for example. For example, the composition may be one comprising a combination of a glucose-rich algae-derived component and a proteinogenic amino acid-rich algae-derived component. The composition for culturing animal cells to be provided by the invention may also be one comprising an algae extract obtained by an extraction method other than with a solid acid catalyst, such as ultrasonic treatment, or a method using a liquid acid (International Patent Publication No. WO2021/066113, for example).

In the method of culturing cells of the invention, the medium to which the composition containing algae-derived components is added may be selected as appropriate for the type of cells to be cultured, but a medium with buffering action is preferred, and for example, it may be phosphate buffer, Eagle medium, Dulbecco's Modified Eagle Medium (DMEM), DMEM:F12 medium, Glasgow Minimum Essential Medium, Grace's Insect Medium, Ham's Medium, Iscove's Modified Eagle Medium, RPMI-1640 medium, L-15 medium or McCoy's 5A Medium, used as a medium that is substantially free of serum and/or added factors for serum-free culture. It may also be one that is substantially free of glucose, vitamins and/or amino acid constituents of proteins (such as one or more amino acids selected from the group consisting of L-glutamine, L-arginine, L-cystine, glycine, L-histidine, L-isoleucine, L-leucine, L-lysine, L-methionine, L-phenylalanine, L-serine, L-threonine, L-tryptophan, L-tyrosine and L-valine). The culture medium may be liquid medium or solid medium. Addition of an algae extract obtained by the method of the invention to an animal cell culturing medium allows nutrients necessary for cell survival (such as growth factors, glucose, amino acid constituents of proteins, and vitamins) to be supplied for cell culturing. The proportion in which the algae extract is added to the animal cell culturing medium may be adjusted as appropriate for the type of cells to be cultured, and for example, the algae extract may be present at 0.1 to 99% (v/v), 1 to 80% (v/v), 2 to 50% (v/v), 3 to 30% (v/v) or 5 to 25% (v/v), for example, with respect to the total volume of the animal cell culturing medium to which the algae extract is added.

According to one embodiment, the invention provides a method for producing an algae extract for use in preparation of a composition for culturing animal cells, which is substantially free of serum and/or added factors for serum-free culture, wherein the method includes:
mixing algae with a solid acid catalyst and heating the mixture to obtain an algae extract.

According to another embodiment, the invention provides a method for producing an algae extract for use in preparation of a composition for culturing animal cells, wherein the method includes:
mixing algae with a solid acid catalyst and heating the mixture to obtain an algae extract.

The scope of the invention also includes a method for producing an algae extract that is expected to be used for preparation of a composition for culturing animal cells that is substantially free of serum and/or added factors for serum-free culture. Thus, the step of mixing the algae extract provided by the invention with an animal cell culturing medium may be carried out by the same person as the person carrying out the invention, or by a different person from the person carrying out the invention.

According to another embodiment, the algae extract provided by the invention may be provided as a kit in which it is separately packaged from the animal cell culturing medium which is substantially free of serum and/or added factors for serum-free culture.

According to yet another aspect, the invention provides a recycling culture method for algae and animal cells. This method may include:
(i) culturing algae using a waste medium obtained from culturing animal cells (first waste medium);
(ii) collecting the algae and mixing and heating with a solid acid catalyst to obtain an algae extract;
(iii) adding the algae extract to the waste medium obtained from culturing the algae of step (i) (second waste medium) and adjusting the concentration of the algae extract to produce a composition for culturing animal cells; and
(iv) using the composition for culturing animal cells for culturing of animal cells.

According to the invention, media from culturing of animal cells (waste medium), which are produced in large amounts in animal cell culturing steps in the fields of regenerative medicine or biomedicine or in the field of cultured foods, and which have previously been treated as waste, can be utilized for culturing of algae (such as microalgae). A medium (waste medium) from culturing of algae may also be used for culturing of animal cells. This allows a recycling culture method for algae and animal cells to be established, in order to help contribute to a lower environmental impact.

In step (i), which is "culturing algae using a waste medium obtained from culturing animal cells (first waste medium)", the first waste medium (also referred to herein simply as "waste medium" for convenience, without meaning that it is actually discarded) may use a medium that can be normally used for culturing of animal cells, and that is a medium obtained from culturing of animal cells. For use according to the invention, the medium for animal cells before culturing of animal cells is not limited to a publicly known type, but may be Eagle medium, Dulbecco's Modified Eagle Medium (DMEM), DMEM:F12 medium, Glasgow Minimum Essential Medium, Grace's Insect Medium, Ham's Medium, Iscove's Modified Eagle Medium, RPMI-1640 medium, L-15 medium, McCoy's 5A Medium or M199 medium, or any medium that allows culturing of animal cells.

The animal cell culturing method in which the first waste medium to be used for the invention is supplied may be any publicly known culturing method (for example, 37°C, saturated steam, humidified atmosphere of 5% CO₂), without any particular restrictions.

For step (ii), which is "collecting the algae, mixing it with a solid acid catalyst and heating the mixture, to obtain an algae extract", the explanation for step (1) of the method for producing a composition for culturing animal cells also applies.

For step (iii), which is "adding the algae extract to the waste medium obtained from culturing the algae of step (i) (second waste medium) and adjusting the concentration of the algae extract to produce a composition for culturing animal cells", the explanation for step (2) of the method for producing a composition for culturing animal cells also applies. In step (iii), however, the waste medium obtained from culturing the algae of step (i) (second waste medium) may be applied instead of the animal cell culturing medium. The composition for culturing animal cells prepared in this step may be used for culturing of animal cells. A recycling culture method for algae and animal cells is established by carrying out these methods.

According to another embodiment, algae may be cultured using the waste medium obtained from culturing animal cells in step (iv) as the first waste medium of step (i), or in other words, step (i) may be carried out again. Steps (i) to (iv) may also be repeated any number of times.

### EXAMPLES

The present invention will now be explained in greater detail by Examples, with the understanding that the invention is not limited in any way by the Examples.

### <Example 1>

### 1. Materials and Methods

### 1-1. Microalgae culturing

For this experiment there was used the eukaryotic microalgae *Chlorella vulgaris* Beijerinck (hereunder, *"Chlorella")* (NIES-2170, National Institute for Environmental Studies, Ibaraki Prefecture). Culturing of C. *vulgaris* was carried out using a mixed medium of Gamborg B5 medium (FujiFilm-Wako Pure Chemical Industries, Osaka Prefecture) and 0.1% Hyponex (HYPONeX Japan, Tokyo). The culturing was carried out using four 1 L screw cap medium bottles (As One Corp., Osaka Prefecture), at 60 rpm with a 4-row low-speed stirrer (As One Corp.), for 24 hours under lighted conditions (LC-LED 450W, Tietech Co., Ltd., Saitama Prefecture) in a room temperature (25°C) environment and with a CO₂ gas concentration of 20%, using a gas exchanger (Tokai Hit, Shizuoka Prefecture) at a gas flow rate of 50 mL/min. After 7 days of culturing, the microalgae were collected using a centrifuge (Sorvall Xpro Series Centrifuge, Thermo-Fischer Scientific, Massachusetts, U.S.) (1700 × g, 10 min), and after centrifugal washing twice with purified water (1700 × g, 5 min), they were collected into a 50 mL screw bottle (As One Corp.). Drying was then carried out with a freezing dryer (FDU-1200, Tokyo Rika, Tokyo).

### 1-2. Nutrient extraction from microalgae

The nutrients were extracted from the microalgae by hydrolysis (hereunder referred to as "solid acid treatment" and "liquid acid treatment"). The weights of the dried algae were measured, and when using a solid acid, treatment was carried out under the conditions shown in Table 1. Specifically, freeze-dried *Chlorella* (100 or 200 mg) and 200 mg of a solid acid (ZP150DH, Futamura Chemical Co., Ltd., Aichi, Japan) were combined, distilled water (4 mL) was added, and the mixture was heated and stirred at the predetermined temperature. After the reaction, the solid portion was removed by filtration and the filtrate was centrifuged for 5 minutes at 1700 × g, using the supernatant for cell culture experimentation.

In the case of liquid acid treatment, freeze-dried *Chlorella* was prepared to 50 g/L and a heat block (Labnet International Inc., New Jersey, U.S.) was used with 1 N HCl at 100°C for 24 hours for heating. After heat treatment, the mixture was neutralized with sodium hydroxide. The solid portion was removed by filtration and the filtrate was centrifuged for 5 minutes at 1700 × g, using the supernatant for cell culture experimentation.

For treatment using ultrasonic waves, the freeze-dried *Chlorella* was prepared to 50 g/L and an ultrasonic cell disruptor (Bioruptor UCD-200TM, Cosmo Bio Co. Ltd., Tokyo) was used for extraction. The extraction was performed with an output of 20 kHz and a treatment time of 10 minutes. The solid portion was removed by filtration and the filtrate was centrifuged for 5 minutes at 1700 × g, using the supernatant for cell culture experimentation.

**Table 1]**

| | Algae species | Algae weight (mg) | Solid acid (mg) | Temperature (°C) | Time (hr) |
|---|---|---|---|---|---|
| Extraction conditions 1 | *Chlorella* | 200 | 200 | 130 | 3 |
| Extraction conditions 2 | *Chlorella* | 100 | 200 | 130 | 3 |
| Extraction conditions 3 | *Chlorella* | 100 | 200 | 160 | 3 |

### 1-3. Cell culturing and cell viability assay

Primary bovine myoblasts were isolated from cow cheek purchased from Tokyo Shibaura Zouki Co., Ltd. The primary bovine myoblasts were cultured with Dulbecco's Modified Eagle Medium (DMEM) (Sigma-Aldrich, Missouri, U.S.) supplemented with 10% fetal bovine serum (FBS, Thermo-Fischer Scientific, Massachusetts, U.S.) and 1% penicillin-streptomycin (P/S, Invitrogen, Carlsbad, California, U.S.), at 37°C under a moist atmosphere containing 5% CO₂. The relative viable cell count was evaluated by XTT assay (Biological Industries, Connecticut, U.S.) and microscopy of the cells. The bovine myoblasts were inoculated into a 96-well plate (AGC Techno Glass Co., Ltd., Shizuoka Prefecture, Japan) at a density of 30,000 cells/well, and cultured overnight in DMEM supplemented with 10% FBS and 1% P/S. After overnight culturing, the medium was discarded and washing was performed twice with phosphate buffered saline (PBS, Sigma-Aldrich). The cells were then cultured for 3 days using samples of 10% FBS-containing DMEM, bovine serum-free DMEM, nutrient-deficient medium and algae extract-added nutrient-deficient medium. In order to eliminate the difference in absorbance by the pigment in each solution, each solution was used with addition of 100 µL of 10% FBS and 1% P/S and 50 µL of XTT reagent, just before starting the XTT assay. The subsequent cell viability assay was carried out according to protocol. Glucose-free DMEM (Invitrogen) or an inorganic salt medium containing only the inorganic salt components of DMEM were prepared in-house for use as nutrient-deficient media. Phase contrast images of the animal cells were taken with a microscope (ELIPSE TS2, Nikon Corp., Tokyo, Japan) and analyzed using software (NIS-Elements BR, Nikon Corp.).

The media used in the Examples had the following compositions unless otherwise specified. In terms of FBS, either 10% FBS-containing or FBS-free medium was used.
(A) Nutrient and mineral composition of DMEM used for ordinary animal cell culturing
   (i) Sugars
      · Glucose: 1000 mg/L or 4500 mg/L
      · Pyruvic acid: 110 mg/L
   (ii) Amino acids
      · L-arginine: 84 mg/L
      · L-cystine: 48 mg/L
      · L-glutamine: 584 mg/L
      · Glycine: 30 mg/L
      · L-histidine: 42 mg/L
      · L-isoleucine: 105 mg/L
      · L-leucine: 105 mg/L
      · L-lysine: 146 mg/L
      · L-methionine: 30 mg/L
      · L-phenylalanine: 66 mg/L
      · L-serine: 42 mg/L
      · L-threonine: 95 mg/L
      · L-tryptophan: 16 mg/L
      · L-tyrosine: 71 mg/L
      · L-valine: 94 mg/L
   (iii) Vitamins
      · Pantothenic acid: 4 mg/L
      · Choline chloride: 4 mg/L
      · Folic acid: 4 mg/L
      · *i*-Inositol: 7.2 mg/L
      · Niacinamide: 4 mg/L
      · Pyridoxine: 4 mg/L
      · Riboflavin: 0.4 mg/L
      · Thiamine: 4 mg/L
   (iv) Minerals
      · CaCl₂: 200 mg/L
      · KCl: 400 mg/L
      · Fe(NO₃)₃-9H₂O: 0.10 mg/L
      · MgSO₄: 98 mg/L
      · NaCl: 6400 mg/L
      · NaHCO₃: 3700 mg/L
      · NaH₂PO₄: 109 mg/L
      · Phenol red: 15 mg/L
(B) Medium composition used in animal cell culture experiment with algae extract
   (1) DMEM (glucose-free)
      Algae extract was used to demonstrate its suitability as a substitute for sugars (glucose/pyruvic acid).
      (i) Sugars
         · Glucose: 0 mg/L
         · Pyruvic acid: 0 mg/L
      (ii) Amino acids
         · L-arginine: 84 mg/L
         · L-cystine: 48 mg/L
         · L-glutamine: 584 mg/L
         · Glycine: 30 mg/L
         · L-histidine: 42 mg/L
         · L-isoleucine: 105 mg/L
         · L-leucine: 105 mg/L
         · L-lysine: 146 mg/L
         · L-methionine: 30 mg/L
         · L-phenylalanine: 66 mg/L
         · L-serine: 42 mg/L
         · L-threonine: 95 mg/L
         · L-tryptophan: 16 mg/L
         · L-tyrosine: 71 mg/L
         · L-valine: 94 mg/L
      (iii) Vitamins
         · Pantothenic acid: 4 mg/L
         · Choline chloride: 4 mg/L
         · Folic acid: 4 mg/L
         · *i*-Inositol: 7.2 mg/L
         · Niacinamide: 4 mg/L
         · Pyridoxine: 4 mg/L
         · Riboflavin: 0.4 mg/L
         · Thiamine: 4 mg/L
      (iv) Minerals
         · CaCl₂: 200 mg/L
         · KCl: 400 mg/L
         · Fe(NO₃)₃-9H₂O: 0.10 mg/L
         · MgSO₄: 98 mg/L
         · NaCl: 6400 mg/L
         · NaHCO₃: 3700 mg/L
         · NaH₂PO₄: 109 mg/L
         · Phenol red: 15 mg/L
   (2) Inorganic salt medium
      Algae extract was used to demonstrate its suitability as a substitute for sugars (glucose/pyruvic acid), amino acids (L-arginine, L-cystine, L-glutamine, glycine, L-histidine, L-isoleucine, L-leucine, L-lysine, L-methionine, L-phenylalanine, L-serine, L-threonine, L-tryptophan, L-tyrosine, L-valine) and vitamins (pantothenic acid, choline chloride, folic acid, i-inositol, niacinamide, pyridoxine, riboflavin and thiamine).
      (i) Sugars
         · Glucose: 0 mg/L
      (ii) Amino acids
         · L-arginine: 0 mg/L
         · L-cystine: 0 mg/L
         · L-glutamine: 0 mg/L
         · Glycine: 0 mg/L
         · L-histidine: 0 mg/L
         · L-isoleucine: 0 mg/L
         · L-leucine: 0 mg/L
         · L-lysine: 0 mg/L
         · L-methionine: 0 mg/L
         · L-phenylalanine: 0 mg/L
         · L-serine: 0 mg/L
         · L-threonine: 0 mg/L
         · L-tryptophan: 0 mg/L
         · L-tyrosine: 0 mg/L
         · L-valine: 0 mg/L
      (iii) Vitamins
         · Pantothenic acid: 0 mg/L
         · Choline chloride: 0 mg/L
         · Folic acid: 0 mg/L
         · *i*-Inositol: 0 mg/L
         · Niacinamide: 0 mg/L
         · Pyridoxine: 0 mg/L
         · Riboflavin: 0 mg/L
         · Thiamine: 0 mg/L
      (iv) Minerals
         · CaCl₂: 200 mg/L
         · KCl: 400 mg/L
         · Fe(NO₃)₃-9H₂O: 0.10 mg/L
         · MgSO₄: 98 mg/L
         · NaCl: 6400 mg/L
         · NaHCO₃: 3700 mg/L
         · NaH₂PO₄: 109 mg/L
         · Phenol red: 15 mg/L

### 2. Results

### 2-1. Cell culturing with microalgae extract

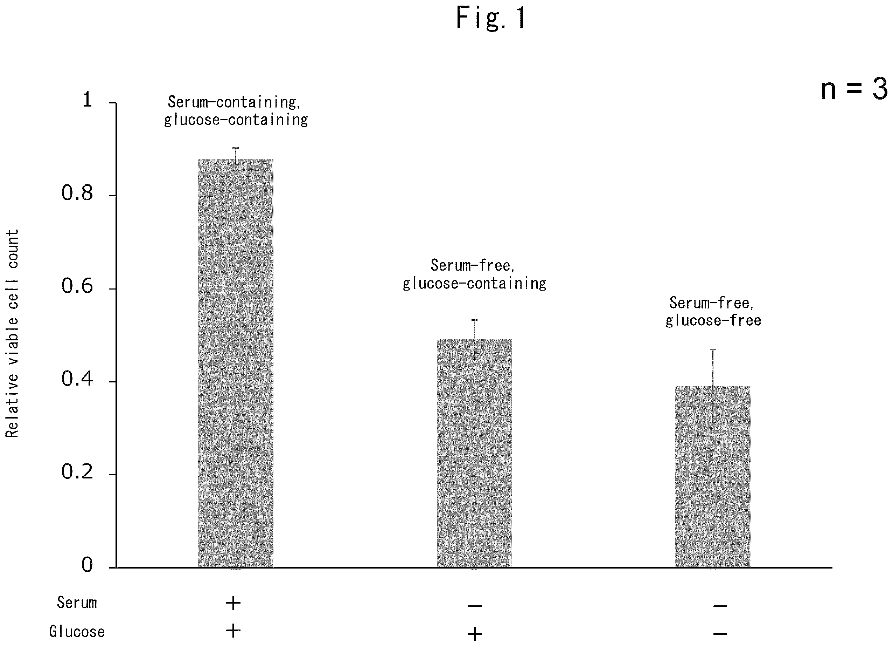

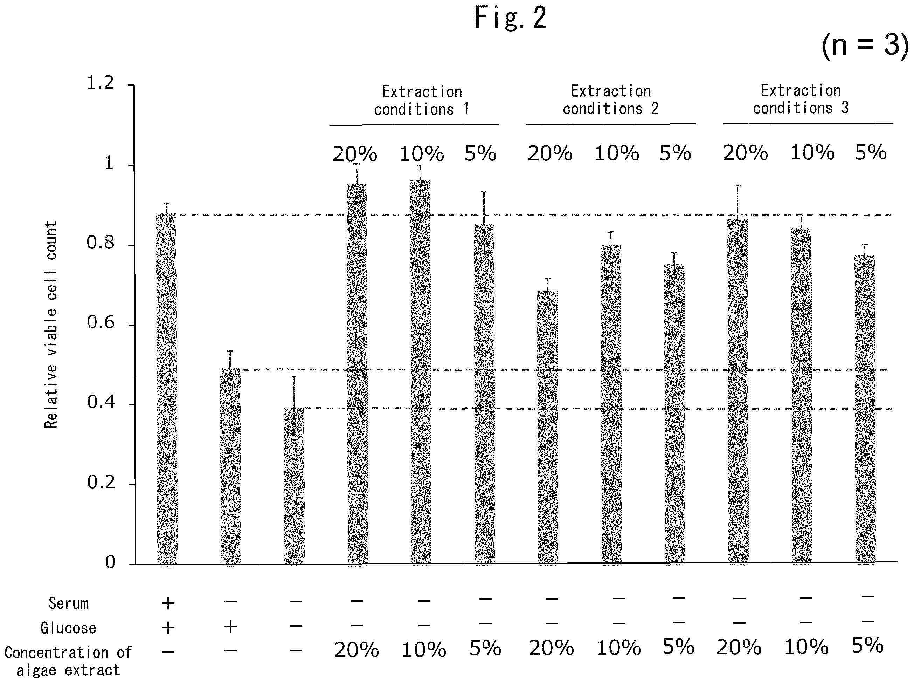

It was next examined whether or not algae extract can be utilized as a nutrient in bovine myoblast culturing. Bovine myoblasts cultured using glucose and/or serum-free medium all had lower cell survival counts compared to culturing with nutrient-containing medium for 3 days (Fig. 1). However, the cell viability recovered by addition of 5%, 10% and 20% extract from C. *vulgaris* using a solid acid (Fig. 2), and a still higher relative viable cell count was exhibited when culturing using nutrient-containing medium. The effect was highest under extraction conditions 1 (130°C, 3 hours). The results verified that algae extract is useful as a substitute for glucose and bovine serum which are important nutrients for culturing of mammalian cells (Fig. 2).

In order to search for more optimal conditions for methods for producing algae extract using a solid acid, microalgae were hydrolyzed under each of the following conditions, and the obtained algae extracts were used for culturing of bovine myoblasts for 3 days.

**Table 2]**

| | Algae species | Algae weight (mg) | Solid acid (mg) | Temperature (°C) | Time (hr) |
|---|---|---|---|---|---|
| Extraction conditions 1 | *Chlorella* | 200 | 200 | 130 | 3 |
| Extraction conditions 4 | *Chlorella* | 100 | 200 | 70 | 6 |
| Extraction conditions 5 | *Chlorella* | 100 | 200 | 70 | 20 |
| Extraction conditions 6 | *Chlorella* | 100 | 200 | 100 | 3 |
| Extraction conditions 7 | *Chlorella* | 100 | 200 | 100 | 6 |

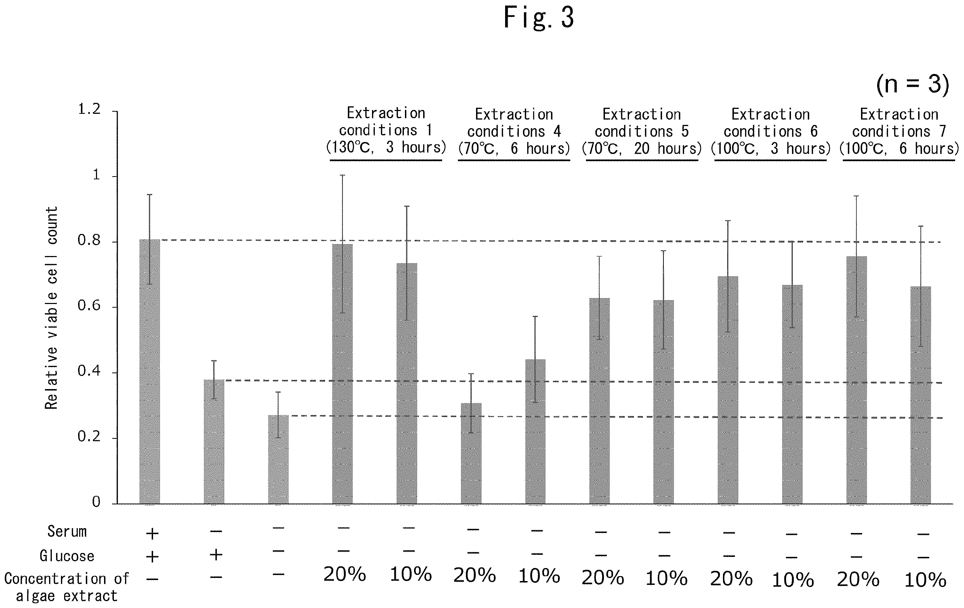

As a result, the algae extract obtained by hydrolysis under extraction conditions 7 (100°C, 6 hours) exhibited the same level of cell proliferation as the algae extract obtained under extraction conditions 1 (130°C, 3 hours) (Fig. 3).

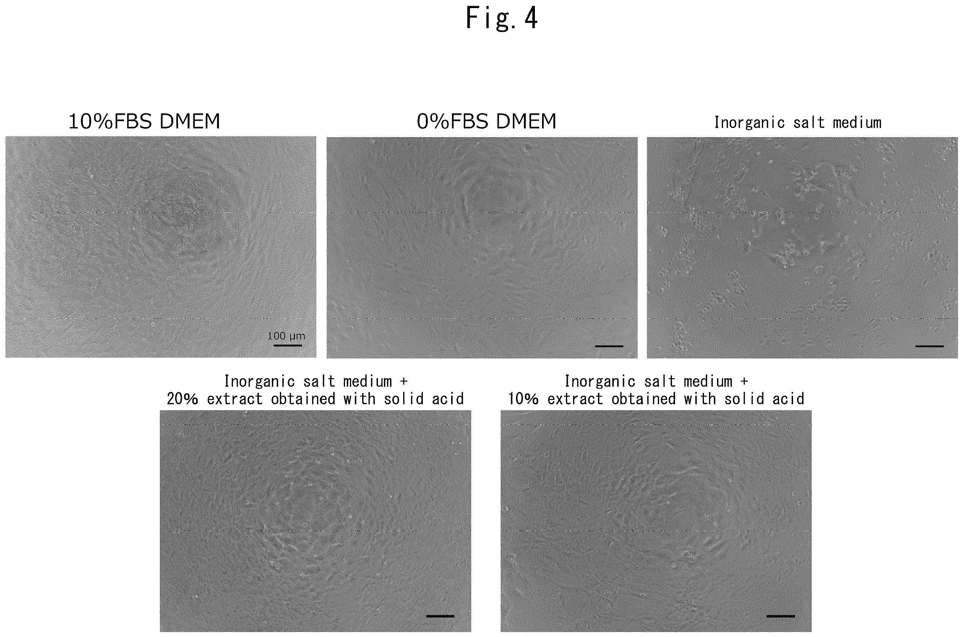

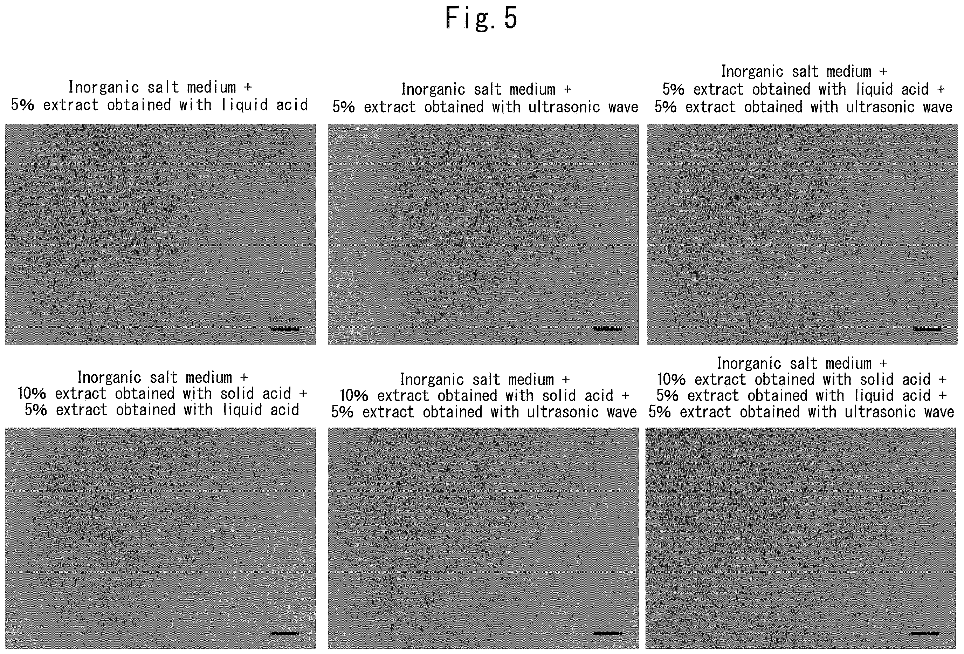

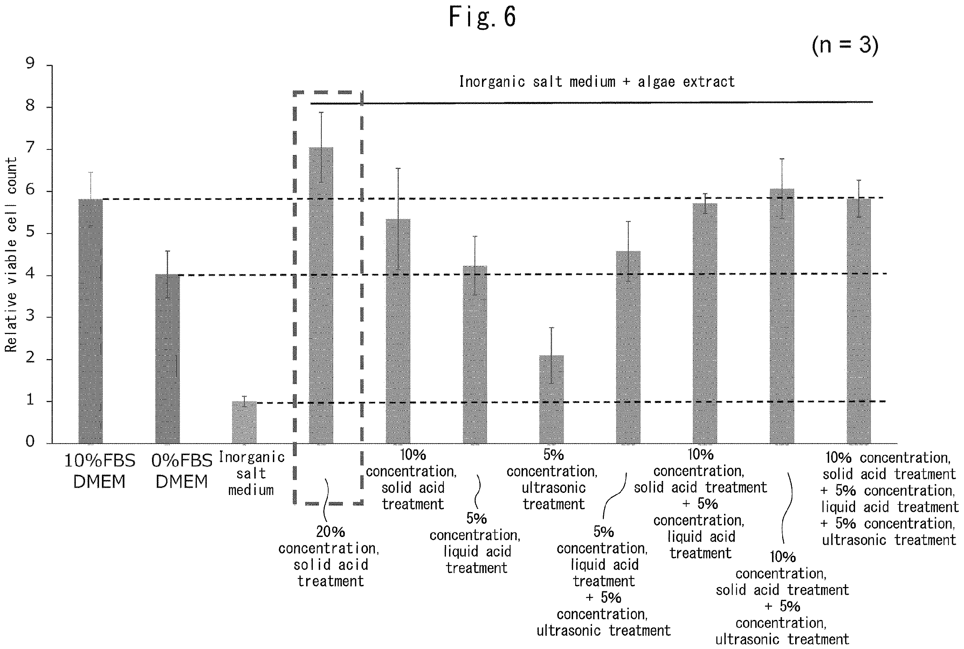

It was also examined whether or not algae extracts can be used as a substitute for both nutrients and animal serum. Inorganic salt medium without nutrients and serum was used. Most of the cells died after culturing for 3 days using this medium (Figs. 4 and 6). However, addition of 20% and 10% solid acid-treated algae liquid extracts produced viable cell counts higher than when culturing with bovine serum-free DMEM and 10% bovine serum-containing DMEM (Figs. 4 and 6). These results indicated that using solid acid-treated algae liquid extracts with inorganic salt components can substitute for nutrients and animal serum necessary for culturing of animal cells. Addition of liquid acid-treated algae extract to inorganic salts, however, produced viable cell counts equivalent to bovine serum-free DMEM (Figs. 5 and 6). The effect of addition of ultrasonically treated algae extract, however, was not sufficient to produce the viable cell count obtained when using bovine serum-free DMEM (Figs. 5 and 6).

### <Example 2>

In regard to the algae extract concentration, the relative viable cell count was generally greater with higher concentration, as shown in Fig. 2, Fig. 3 and Fig. 6. This was not necessarily always the case, however, being dependent on the extraction conditions and type of algae. When constructing the following recycling system, therefore, it is necessary to set the concentration of the extract based on component analysis for extraction conditions and medium that yield the maximal viable cell count.

Addition of algae extract to an animal cell culturing medium was thus shown to have a major effect on culturing of animal cells, even when the medium was substantially free of serum or glucose. The present inventors have already demonstrated that culturing of algae is possible using waste medium from animal cell culturing (Haraguchi Y, Shimizu T. Microalgal culture in animal cell waste medium for sustainable 'cultured food' production. Arch. Microbiol. 2021 Nov; 203(9):5525-5532.).

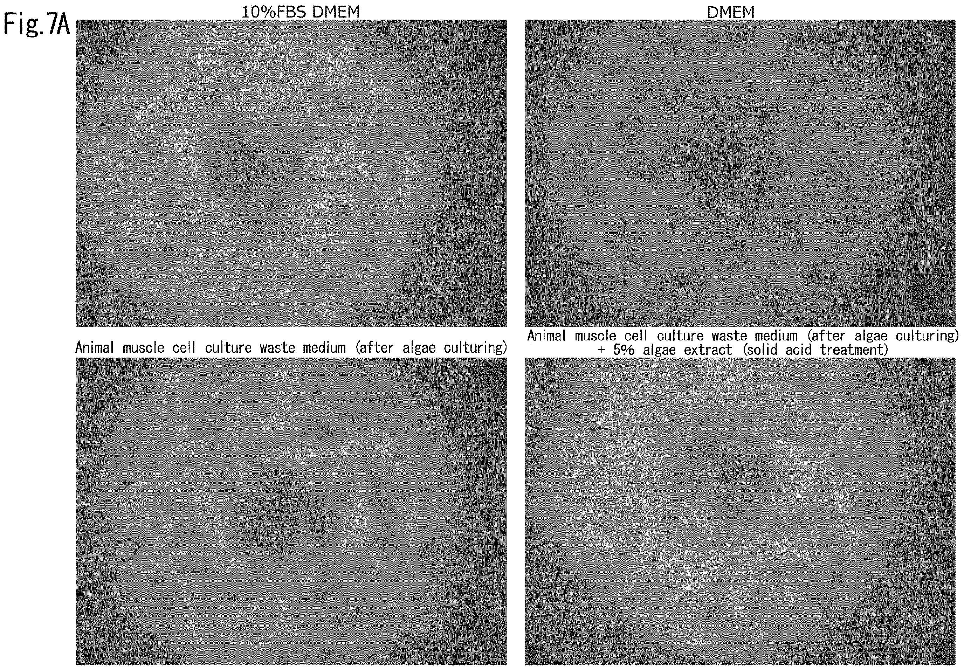

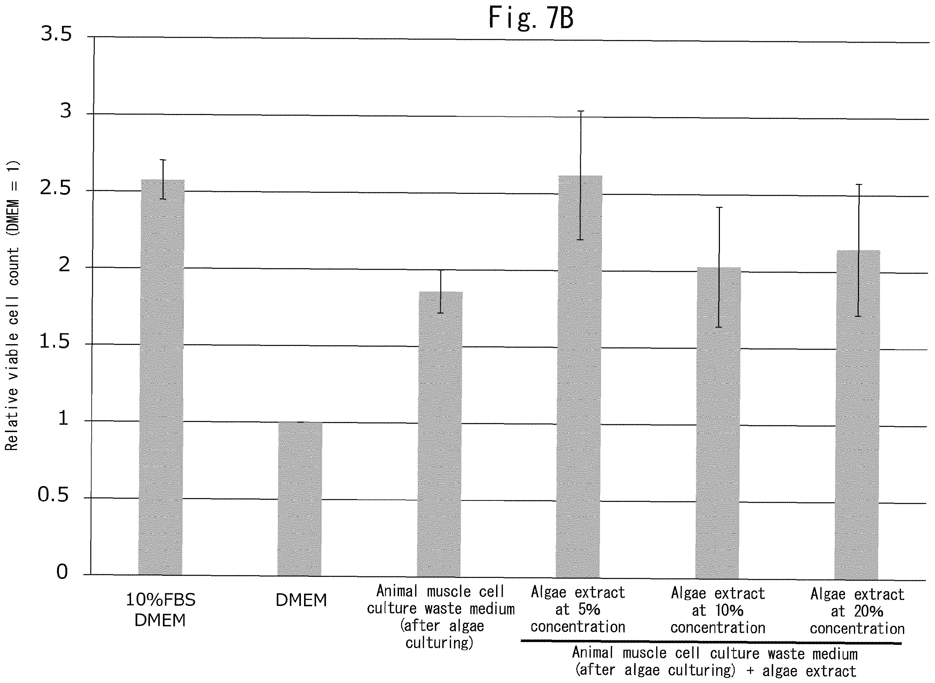

It was shown that culturing of animal cells (C2C12 mouse myoblasts) is possible when extract from algae (*Chlorococcum littorals*) obtained by the method of the invention is added to culture waste medium obtained from culturing *Chlorococcum littorale* for 2 days (second waste medium) using a culture waste medium obtained from culturing animal cells (C2C12 mouse myoblasts) for 2 days (first waste medium) (Fig. 7, A: post-culturing photomicrograph, B: quantitative evaluation of relative viable cell count). It was thus demonstrated that a recycling system can be constructed in which animal cells are cultured with addition of extract obtained by culturing algae using waste medium from culturing of animal cells and solid acid treatment of the algae, to waste medium (second waste medium) obtained from culturing of algae using an animal cell waste medium (first waste medium).

## Claims

1. A method for producing a composition for culturing animal cells, comprising the following steps:
(1) mixing algae with a solid acid catalyst and heating the mixture to obtain an algae extract; and
(2) adding the algae extract to animal cell culturing medium and adjusting the concentration of the algae extract.

2. The method according to claim 1, wherein the animal cell culturing medium is substantially free of serum and/or added factors for serum-free culture.

3. The method according to claim 1 or 2, wherein the animal cell culturing medium is substantially free of glucose.

4. The method according to any one of claims 1 to 3, wherein the animal cell culturing medium is substantially free of one or more amino acids selected from the group consisting of L-glutamine, L-arginine, L-cystine, glycine, L-histidine, L-isoleucine, L-leucine, L-lysine, L-methionine, L-phenylalanine, L-serine, L-threonine, L-tryptophan, L-tyrosine and L-valine.

5. The method according to any one of claims 1 to 4, wherein the animal cell culturing medium is substantially free of vitamins.

6. The method according to any one of claims 1 to 5, wherein the animal cell culturing medium is an inorganic salt medium.

7. The method according to any one of claims 1 to 6, wherein the algae are microalgae.

8. The method according to any one of claims 1 to 7, wherein the algae are microalgae belonging to *Chlorella* sp..

9. The method according to any one of claims 1 to 8, wherein the solid acid catalyst is a woody solid acid catalyst obtained by introducing a sulfo group into a carbide derived from a wood-based raw material for sulfonation or a resin solid acid catalyst obtained by introducing a sulfo group into a phenol resin for sulfonation.

10. The method according to any one of claims 1 to 9, wherein step (1) is carried out at 70°C to 150°C.

11. The method according to any one of claims 1 to 10, wherein step (1) is carried out for 2 hours to 24 hours.

12. A composition for culturing animal cells which is obtained by the method according to any one of claims 1 to 11.

13. A method for culturing animal cells, using a composition for culturing animal cells which is obtained by the method according to any one of claims 1 to 11.

14. A recycling culture method for algae and animal cells, comprising:
(i) culturing algae using a waste medium obtained from culturing animal cells (first waste medium);
(ii) collecting the algae and mixing and heating with a solid acid catalyst to obtain an algae extract;
(iii) adding the algae extract to a waste medium obtained from culturing the algae of step (i) (second waste medium) and adjusting the concentration of the algae extract to produce a composition for culturing animal cells; and
(iv) using the composition for culturing animal cells, for culturing of animal cells.

15. A method for producing an algae extract for use in preparation of a composition for culturing animal cells, comprising:
(1) mixing algae with a solid acid catalyst and heating the mixture to obtain an algae extract.
